# FASCICULE DE BREVET EUROPEEN

(11) **EP 0 655 928 B1**
(45) Date de publication et mention de la délivrance du brevet: **29.12.1997**
(21) Numéro de dépôt: 92918959.5
(22) Date de dépôt: 21.08.1992
(51) Int. Cl.: A61K 31/00, A61K 33/00, A61K 35/00

(54) **Utilisation d'un produit homéopathique pour la préparation d'un médicament destiné au traitement de maladies métaboliques**
Verwendung eines homöopathisches Erzeugnisses zur Herstellung eines Arzneimittels für die Behandlung von metabolischen Krankheiten
Use of an homeopathic product for the manufacture of a medicament for treatment of metabolic diseases

(43) Date de publication de la demande: 07.06.1995
(73) Titulaire: ETIENNE, Marie-Christine, F-69160 Tassin-la-Demi-Lune (FR)
(72) Inventeur: ETIENNE, Marie-Christine, F-69160 Tassin-la-Demi-Lune (FR)
(86) Numéro de dépôt international: FR9200813
(87) Numéro de publication internationale: WO9404186

(56) Documents cités:
- EP-A- 0 315 552
- G.NETIEN ET AL 'galenica 16 médicaments homéopathiques' 1986 , TECHNIQUE ET DOCUMENTATION , PARIS, FR

## Description

La présente invention concerne l'utilisation d'un produit homéopathique pour l'obtention d'un médicament destiné au traitement de maladies métaboliques.

Les maladies métaboliques concernées sont des maladies caractérisées par l'accumulation intracellulaire ou par la carence intracellulaire en un corps chimique de formule simple ou complexe, variable selon le cas et désigné ici par R.

R agit en fait sur les systèmes transporteurs péricellulaires vis-à-vis de lui-même, qui s'étaient détraqués et dont il rétablit un fonctionnement correct. Ces maladies, sont souvent appelées génétiques, dans l'état de l'art antérieur qui avoue son impuissance à les guérir.

Les remèdes homéopathiques sont connus comme étant définis par une dilution et une dynamisation. Dans l'état antérieur de la technique, l'utilisation des remèdes homéopathiques repose sur la loi de similitude, le principe de la similitude, le principe de l'infinitésimalité et sur les pathogénésies.
- La loi de similitude est la suivante : "Un produit quelconque qui administré à forte dose déclenche certains troubles chez l'homme en bonne santé, devient à dose très faible c'est-à-dire après dilution homéopathique, le remède capable de guérir ces mêmes troubles chez l'homme malade". La loi de similitude consiste donc à donner au malade à dose infinitésimale c'est-à-dire en dilution homéopathique la substance qui administrée à un sujet sain provoque chez lui des symptômes semblables à ceux du malade.
- Le principe de la similitude est le suivant :
   "Tout organisme malade présente un ensemble de symptômes qui représente la maladie.
   Toute substance développe dans l'organisme des symptômes qui lui sont propres et qui sont fonction de la substance et de la réceptivité de l'organisme sensible. La guérison, objectivée par la disparition des symptômes morbides peut être obtenue par la prescription d'une substance dont les effets sont semblables à ceux de la maladie observée".

L'utilisation thérapeutique des remèdes homéopathiques dans l'art antérieur consiste donc à faire disparaître chez un individu malade une série de symptômes par la substance qui a pu provoquer chez un sujet sain les mêmes symptômes, en administrant cette substance en dilution homéopathique.
- Le principe de l'infinitésimalité est le suivant :
   Pour qu'une substance soit capable de guérir chez un sujet malade les symptômes qu'elle produit chez un sujet sain, il faut qu'elle soit prescrite à petite dose. Dans l'art antérieur, l'utilisation des remèdes homéopathiques est basée sur la présence d'une petite dose de produit actif au sein de la dilution homéopathique et cette utilisation consiste à stimuler les réactions de l'organisme au moyen de ces petites doses.
- Ainsi, dans l'état de l'art antérieur, pour traiter un malade atteint de mucoviscidose et qui tousse, l'homme de l'art, a à sa disposition plus de 30 remèdes servant à traiter une toux, chacun étant défini par sa pathogénésie c'est-à-dire par l'ensemble des symptômes de toux que la substance contenue dans le remède a pu déterminer chez un sujet antérieurement sain.
- Ainsi, pour traiter une maladie auto-immune, dans l'état de l'art antérieur, on utilise l'organothérapie :
- L'organothérapie consiste à administrer des dilutions homéopathiques de l'organe qui est le siège de la maladie auto-immune. Suivant le principe de l'infinitésimalité les petites doses d'organes contenues dans les dilutions homéopathiques se fixent sur les auto-anticorps circulants. Le complexe formé s'élimine par les voies naturelles et les anticorps anormaux sont ainsi détournés de leur cible.
   (Toutes les citations sont extraites de Galenica - Volume 16 : Médicaments Homéopathiques).

L'invention, telle que caractérisée à la revendication 1, concerne une deuxième utilisation thérapeutique des remèdes homéopathiques qui ne sont plus utilisés suivant la loi de similitude, le principe de la similitude, le principe de l'infinitésimalité et les pathogénésies pour faire disparaître chez un sujet malade une série de symptômes en se servant à dose infinitésimale de la même substance qui a pu provoquer chez le sujet sain à dose pondérale la même série de symptômes, mais qui sont utilisés, pour provoquer une élimination de corps chimiques hors de la cellule et hors de l'organisme dans le but de rétablir le fonctionnement normal de systèmes transporteurs péricellulaires perturbés vis-à-vis de ces corps chimiques.

L'invention se fonde sur une propriété des dilutions homéopathiques connue et expérimentée seulement in vitro : depuis les expériences de Lise WURMSER (reprises depuis par de nombreux auteurs), l'on a constaté qu'un produit en dilution homéopathique provoquait l'élimination hors de l'organisme du produit à dose pondérale à partir duquel il avait été préparé, le produit éliminé se trouvant stocké à l'intérieur des cellules ; le terme produit désignant ici n'importe quel corps chimique. Lise WURMSER et ses collaborateurs ont injecté de l'arsenic à des animaux de laboratoire, puis ont injecté à ces cobayes de l'arsenic en dilution homéopathique. Lise WURMSER a montré que sous l'influence des dilutions homéopathiques d'arsenic, il était constaté une élimination accrue dans les urines et dans les selles des cobayes de l'arsenic injecté. Ces expériences ont été reprises par la suite par de nombreux auteurs, notamment avec le plomb, l'antimoine et encore avec l'arsenic.

Des observations cliniques originales effectuées par l'auteur de la présente invention lui ont démontré que cette propriété expérimentale d'élimination pouvait aussi être utilisée lors de prescriptions cliniques de dilutions homéopathiques. L'invention consiste à avoir apporté une conclusion originale et nouvelle aux travaux de Lise WURMSER et cette conclusion est la suivante : que la dilution homéopathique d'arsenic a exercé sur l'arsenic stocké à l'intérieur des cellules une attraction électromagnétique suffisamment forte pour le faire sortir hors des cellules, que le résultat de cette attraction a été l'obtention d'un composé électromagnétiquement neutre qui a été éliminé ensuite par les voies naturelles : l'urine et les selles. Selon l'invention cette propriété d'attraction électromagnétique inverse se constitue lors de la fabrication des remèdes homéopathiques au cours de la dynamisation et l'attraction électromagnétique est d'autant plus forte que la dilution est plus élevée et que par conséquent Il y a eu plus de dynamisations. Selon l'invention plus la dilution homéopathique est élevée et donc plus il y a eu de dynamisations concomitantes, plus la puissance éliminatrice est forte : c'est ainsi qu'une 30CH possède selon l'invention une puissance éliminatrice plus forte qu'une 15CH, qui elle a une puissance éliminatrice plus forte qu'une 5CH.

Dans l'état de l'art antérieur, l'utilisation des remèdes homéopathiques suivant la loi de similitude est basée sur la présence d'une petite dose de produit actif au sein des dilutions et ce sont les basses dilutions qui contiennent le plus de produit actif et qui sont par conséquent les plus efficaces.

Il a été constaté par des observations cliniques originales que cette élimination d'éléments intracellulaires hors de la cellule et hors de l'organisme peut influencer le fonctionnement des systèmes transporteurs péricellulaires de façon durable. L'Invention se fonde sur l'utilisation de cette propriété d'élimination de corps chimiques intracellulaires hors de la cellule et hors de l'organisme sous l'influence de leurs dilutions homéopathiques pour rétablir un fonctionnement normal des systèmes transporteurs péricellulaires perturbés.

Dans l'état antérieur de la technique les maladies dues à des anomalies de fonctionnement des systèmes transporteurs péricellulaires sont qualifiées de génétiques. Parmi les anomalies de fonctionnement de ces systèmes transporteurs, seules sont connues dans l'état antérieur de la technique les anomalies de fonctionnement des canaux ioniques et parmi les canaux ioniques seuls sont connus les canaux Sodium, Potassium, Calcium et Chlore. L'invention a pour origine la déduction de constatations cliniques originales portant sur des prises de remèdes homéopathiques, que les passages de corps chimiques de l'extérieur vers l'intérieur de la cellule et vice versa, sont dus à de multiples systèmes transporteurs péricellulaires et que ceux-ci fonctionnent suivant les lois de l'osmose faisant passer les corps chimiques du milieu le plus concentré vers le milieu le moins concentré. Selon la présente invention les canaux ioniques sont un cas particulier de ces systèmes transporteurs de corps chimiques.

Jusqu'à présent, dans l'état de l'art antérieur, l'on utilisait pour modifier le fonctionnement des canaux ioniques et des transports d'ions de l'extérieur vers l'intérieur de la cellule des substances chimiques à dose pondérale (c'est-à-dire non homéopathique), le plus souvent protéiques, qui en se fixant sur certains sites récepteurs (des canaux ioniques par exemple) modifient l'entrée et la sortie d'ions de la cellule. Comme exemple, on cite :
- les Diurétiques :
   . Le Lasilix® (Furosémide) qui au niveau de l'anse de Henlé du rein inhibe la réabsorption du chlore et du sodium.
   . L'Adactone® (Spironolactone) qui se place sur différents récepteurs extrarénaux et sur le tube contourné distal dans les sites d'action de l'aldostérone aux points d'échange Sodium-Potassium, et qui se comporte comme un antagoniste de l'aldostérone en bloquant ses sites récepteurs.
- les inhibiteurs calciques qui se fixent sur des sites récepteurs protéiques dans le muscle lisse et le muscle cardiaque et empèchent l'entrée dans la cellule du Calcium qui y rentrait par le moyen des canaux calciques. Ces inhibiteurs calciques bloquent les canaux calciques lents.

Les recherches s'orientent pour agir sur les canaux ioniques vers des manipulations génétiques : c'est le cas de la mucoviscidose.

Lorsque les systèmes transporteurs concernent d'autres substances que les ions, l'art antérieur ne comprend aucune technique. On mentionne toutefois que dans le cas de surcharge intracellulaire en métaux, dans l'art antérieur, on utilise couramment des chélateurs qui provoquent une élimination de ces métaux hors des cellules et hors de l'organisme.

Dans l'art antérieur, les états de carence ou d'excès intracellulaire en ions sont connus : ces états sont qualifiés de maladies génétiques. Leur origine est rapportée à des anomalies situées sur les chromosomes. Il n'y a que pour le cas de la seule mucoviscidose que l'art antérieur affirme que cette maladie génétique est due à un dysfonctionnement des canaux ioniques péricellulaires : les canaux sodium et chlore.

L'invention peut conduire à affirmer que toutes les maladies par carence ou excès intracellulaire en un corps chimique et que dans l'art antérieur, on qualifie de génétique, sont dues à des dysfonctionnements de systèmes transporteurs péricellulaires vis-à-vis du corps chimique en excès ou en carence à l'intérieur des cellules.

L'invention qui se base sur ces théories nouvelles et originales se fonde sur l'utilisation de la propriété d'élimination de corps chimiques hors de la cellule et hors de l'organisme sous l'influence de leur dilution homéopathique, pour rétablir un fonctionnement normal des systèmes transporteurs péricellulaires perturbés vis-à-vis de ces corps chimiques. Dans le cadre de l'invention, les canaux ioniques et systèmes transporteurs péricellulaires fonctionnent suivant des gradients de concentration faisant passer les ions et autres substances du milieu le plus concentré vers le milieu le moins concentré. Du fait des lois de l'osmose l'eau suit les particules et c'est le milieu où il y a le plus grand nombre de molécules qui devient le milieu le moins concentré de sorte que les systèmes transporteurs se bloquent dans le sens de l'entrée dans la cellule s'il y a déjà un excès du corps chimique considéré dans la cellule ou dans le sens de la sortie de la cellule s'il y a déjà dans la cellule une carence du corps chimique considéré.

Ces systèmes transporteurs se comportent comme une multitude de portes ne fonctionnant que dans un sens, comme les portes d'entrée d'un supermarché qui laissent soit entrer, soit sortir. Ces portes qui laissent passer le corps chimique considéré sont bloquées soit dans le sens de l'entrée dans la cellule lorsqu'il y a excès intracellulaire en ce corps chimique, soit dans le sens de la sortie de la cellule lorsqu'il y a carence intracellulaire en ce corps chimique.

Dans le cadre de l'invention, dans le cas des maladies par rétention intracellulaire, en minéraux par exemple, l'eau passe vers l'intérieur des cellules où les minéraux se trouvent déjà en grande quantité et déconcentre l'intérieur de la cellule où il y a le plus de minéraux. (L'on prend l'exemple non limitatif de minéraux, mais il peut s'agir de rétention intracellulaire en d'autres produits de natures chimiques diverses). Les minéraux ont donc une tendance indéfinie à s'accumuler à l'intérieur de la cellule où ils sont déjà en excès. La propriété d'élimination d'éléments intracellulaires hors de la cellule et hors de l'organisme sous l'influence de leur dilution homéopathique est immédiatement applicable à ce type d'affection. La dilution homéopathique de l'élément en excès provoque une sortie hors des cellules de l'élément (ou du corps chimique quel qu'il soit) en excès, ce qui diminue la surcharge intracellulaire. Cependant, il existe un autre mécanisme expliquant une action sur les systèmes transporteurs péricellulaires : l'administration de la dilution homéopathique du produit en excès dans la cellule provoque aussi un appel d'eau autour des molécules éliminées, car l'eau suit les minéraux et autres produits et sort de la cellule. Le milieu extracellulaire devient ponctuellement moins concentré que le milieu intracellulaire. Les systèmes transporteurs péricellulaires correspondant à cet endroit, qui sont réglés du fait d'un gradient de concentration, c'est-à-dire quelques unes des portes d'entrée basculent pour faire sortir hors de la cellule le produit qui ne pouvait qu'y rentrer. Les conséquences de ces éliminations ponctuelles et répétées de quelques molécules sont un rétablissement à long terme du fonctionnement normal des systèmes transporteurs, ceci sous l'influence de la prise répétée au long des semaines, des mois et des années du remède homéopathique considéré. Les dilutions utilisées selon l'invention, sont de préférence hautes par exemple 15CH à 30CH, car ces hautes dilutions provoquent selon l'invention une élimination plus énergique que les basses dilutions. Leur action se prolonge plusieurs jours de suite, aussi leur prise est espacée de 1 à 2 fois par semaine.

Dans le cadre de l'invention, dans les maladies par carence intracellulaire en un produit quelconque par exemple en un minéral, le milieu intracellulaire carencé et pauvre en molécules d'ions ou d'un autre produit ne contient que peu d'eau, puisque l'eau a afflué vers le secteur extracellulaire riche en ions minéraux (ou en autre produit). L'intérieur de la cellule est devenu plus concentré que l'extérieur de la cellule, et les systèmes transporteurs qui sont réglés suivant un gradient de concentration et font passer du milieu le plus concentré vers le milieu le moins concentré, sont bloqués dans le sens de la sortie.
L'administration dans ce cas du produit en carence intracellulaire, en dilution homéopathique, provoque dans le cadre de l'invention :
- soit une élimination hors de la cellule des quelques molécules du produit ou du minéral restant ce qui fait tomber ponctuellement la concentration intracellulaire à zéro et incite de cette façon un transporteur à basculer dans le sens de l'entrée pour faire rentrer dans la cellule l'ion ou le produit en carence au point où la concentration était zéro.
- soit neutralise des molécules du produit ou du minéral dans le secteur extracellulaire et les fait éliminer en laissant les molécules d'eau qui les entouraient seules. Du fait des lois de l'osmose, pour rétablir une concentration en eau égale de part et d'autre de la membrane cellulaire, ces molécules d'eau rentrent dans la cellule et c'est la cellule que va ponctuellement devenir le milieu le moins concentré. Les portes du système transporteur correspondant basculent et se positionnent dans le sens de l'entrée dans la cellule.

Dans le cadre de l'invention, des prises répétées du minéral ou du produit en carence intracellulaire en dilution homéopathique ont pour conséquence à long terme une bascule d'un grand nombre de systèmes moléculaires transporteurs dans le sens de l'entrée dans les cellules et un rétablissement du fonctionnement normal de ces cellules par rétablissement du fonctionnement normal des systèmes transporteurs péricellulaires. Les dilutions utilisées, dans le cadre de l'invention, sont de préférence basses, à titre d'exemple non limitatif des 4CH, 5CH ou 7CH car ces dilutions provoquent une élimination moins forte que les hautes dilutions. Leur effet est de courte durée et leur prise dans le cadre de l'invention doit être quotidienne.

Dans le cadre de l'invention, le rétablissement du fonctionnement normal des systèmes transporteurs guérit des maladies et anomalies métaboliques secondaires à ces dysfonctionnements, mais où le rapport avec les systèmes transporteurs et les carences ou excès intracellulaires peut être évident et direct, ou être très indirect et pas évident du tout.

Dans le cadre de l'invention, la posologie de la dilution homéopathique est la suivante : l'administration de la dilution homéopathique du produit concerné doit être régulière et peut durer longtemps, souvent plusieurs années. La fréquence des prises et la hauteur de la dilution des composés selon l'invention est une affaire de cas particuliers et doit être déterminée par le thérapeute. La formule générale des composés est RxCH.

A titre d'exemple indicatif et non limitatif l'on donne ci-après une liste d'un certain nombre de corps chimiques qui peuvent être R, selon l'invention :
NaCl (sel marin ou Natrum muriaticum)
Fer Réduit (Ferrum métallicum)
Sulfure noir d'antimoine (Antimonium crudum)
Or (Aurum métallicum)
Potassium
Phosphate dipotassique ou Kalium phosphoricum
Aluminium (Aluminium metallicum)
Phosphate dimagnesien (Magnesia phosphorica)
Phosphate Tricalcique (Calcarea phosphorica)
Cuivre (Cuprum metallicum)
Le calcaire d'huitre (Calcarea carbonica)
Acide oxalique (Oxalic acid)
Oxalate de calcium (Calcarea oxalica)
La Mélanine (Melaninum)
L'encre de Seiche (Sepia)
Ces composés RxCH se présentent sous forme de granules ou de globules ou sous les autres formes (ampoules suppositoires etc...) couramment utilisées en Homéopathie. Les prises peuvent être perlinguales, peros, IM, IV, Ampoule suppositoire ou sous tout autre forme médicinale possible.

Pour rétablir un fonctionnement normal de systèmes transporteurs péricellulaires vis-à-vis d'une carence intracellulaire selon l'invention, il est recommandé d'utiliser x = 5CH une fois par jour en granulés à raison de 3 à 5 granules par jour, ceci à titre indicatif et non limitatif.

Pour rétablir un fonctionnement normal des systèmes transporteurs péricellulaires vis-à-vis d'un excès intracellulaire, il est recommandé, selon l'invention d'utiliser x = 15 CH en dose une fois par semaine ceci à titre indicatif et non limitatif : R15CH.
- Dans le cas des intoxications où lorsque dans le composé RxCH, R est un toxique (ou une fraction du toxique),
   il s'agit d'une élimination du toxique hors de la cellule par action sur des systèmes transporteurs péricellulaires.
- Dans le cas des maladies métaboliques, le rétablissement du fonctionnement normal des systèmes transporteurs péricellulaires guérit des anomalies métaboliques secondaires à ces dysfonctionnements, où le rapport avec les carences ou les excès intracellulaires peut être évident et direct ou très indirect et pas évident du tout.
- Quand il est évident et direct, il s'agit de maladies par excès ou carence intracellulaire en un corps chimique et dans lesquelles les manifestations pathologiques sont la conséquence directe de l'accumulation intracellulaire ou de la carence intracellulaire en ce corps chimique :
   . Par exemple la mucoviscidose qui est une conséquence directe d'un excès intracellulaire en NaCl, par perturbation des systèmes transporteurs péricellulaires vis-à-vis du NaCl.
   . Par exemple la tétanie par carence intracellulaire en magnesium, secondaire à une perturbation des systèmes de transports péricellulaires vis-à-vis du magnésium, et où les symptômes pathologiques de la maladie sont des symptômes de carence en magnésium.
- Quand ce rapport est très indirect et pas évident du tout, il s'agit de maladies par excès ou par carence intracellulaire en un corps chimique dans lesquelles cet excès ou cette carence a pour conséquence l'élaboration par la cellule de susbstances anormales responsables des manifestations pathologiques observées. Ces substances ont une structure qui présente des anomalies qui sont dues aux conditions anormales dans lesquelles la cellule fonctionne et qui sont à l'origine d'affections qui n'ont plus aucun rapport avec l'anomalie des systèmes transporteurs causale : ainsi les cellules se mettent à fabriquer des anticorps de structure anormale responsables d'allergies ou s'il s'agit d'auto-anticorps, ils sont responsables de maladies auto-immunes. Ainsi, secondairement aux anomalies des systèmes transporteurs, les cellules se mettent à fabriquer toutes sortes de produits chimiques de structure anormale ou en quantité anormale, responsables d'affections les plus diverses telles qu'hypertension artérielle s'il s'agit d'une hyperproduction d'aldostérone, goutte s'il s'agit d'une hyperproduction d'acide urique, dyslipémies et athérosclérose s'il s'agit de cholestérol ou de triglycérides en quantité anormalement élevée, et cette liste n'est pas limitative.

Les exemples d'observations cliniques montrent :
- qu'une carence intra-cellulaire par anomalie des systèmes transporteurs péricellulaires vis-à-vis du NaCl aura pour conséquence la production par les cellules d'anticorps anormaux de type IgE responsables d'une rhinite allergique et une hyperproduction d'hormones thyroïdiennes responsable d'une hyperthyroïdie.
- qu'une anomalie des systèmes transporteurs péricellulaires vis-à-vis du sulfure noir d'antimoine a pour conséquence la production par les cellules d'anticorps auto-immuns ou de métabolites inflammatoires responsables d'une polyarthrite chronique rhumatoïde.
- que la carence intracellulaire en or par détraquage des systèmes transporteurs péricellulaires vis-à-vis de l'or peut avoir comme conséquence la production par les cellules d'anticorps auto-immuns.

Toutes ces maladies par dysfonctionnement des systèmes transporteurs, selon l'Invention, sont connues dans l'état antérieur de la technique pour être des maladies génétiques ; et l'on a effectivement décrit des anomalies de certains gènes expliquant les pathologies observées. Selon l'invention la correction des dysfonctionnements des systèmes transporteurs péricellulaires par l'utilisation des composés homéopathiques RxCH corrige aussi les anomalies chromosomiques commandant les dysfonctionnements des systèmes transporteurs péricellulaires et interrompt la transmission génétique de ces maladies. En effet, selon l'invention, la correction du dysfonctionnement des systèmes transporteurs péricellulaires agit sur les anomalies des gènes situées sur les chromosomes en les normalisant aussi. Dans le cadre de l'invention, les anomalies de fonctionnement des systèmes transporteurs péricellulaires peuvent avoir d'autres origines et d'autres causes qu'une transmission héréditaire.

**A titre indicatif et non limitatif on donne ci-après des exemples de maladies métaboliques souvent appelées génétiques à traiter par les composés RxCH constitutifs d'un médicament dont l'invention permet l'obtention.**

### A) Les Maladies par excès intracellulaire :

### Exemple 1 : La mucoviscidose

La mucoviscidose est une maladie dite génétique caractérisée par un excès de chlorure de sodium dans les sécrétions et à l'intérieur des cellules. Certains auteurs rapportent aussi un excès de potassium. Le traitement dans l'art antérieur, n'existe pas. Les recherches portent sur l'hypothèse que l'on pourrait utiliser une protéine appelée CFRT qui empêcherait le NaCl de rentrer dans les cellules, ou que l'on pourrait utiliser des manipulations génétiques. L'invention a pour objet de traiter la mucoviscidose par un composé de formule RxCH dans lequel R peut être à titre indicatif et non limitatif le NaCl, ou tout autre sel susceptible d'agir sur la surcharge ionique considérée et le canal ionique défectueux tels que le chlorure de potassium, l'iodure de sodium, le chlorure d'iode, ou des sels composés du sodium, du chlore, du potassium et de l'iode, comprenant tous ces éléments ou une partie d'entre eux, et où x peut être de préférence égal à 15 ou 30.

### Exemple 2 : La Rétinite pigmentaire

La rétinite pigmentaire est une maladie dite génétique et incurable due à une accumulation de pigments mélaniques ou autres dans les cellules de la rétine et qui aboutit à la cécité. L'invention consiste à traiter cette affection par le composé RxCH où R peut être à titre indicatif la mélanine ou Sepia, produit actif bien connu en homéopathie, qui est de la mélanine mélangée à des impuretés, ou un autre pigment, et où x est de préférence égal à 15 ou 30.

### Exemple 3 : L'oxalose

C'est une maladie dite génétique dans laquelle il y a accumulation intracellulaire d'acide oxalique ou d'oxalate de calcium. Cette affection est observée chez l'enfant et aboutit à la mort par insuffisance rénale. L'invention consiste à traiter l'oxalose par le composé RxCH dans lequel R peut être de préférence : l'acide oxalique ou l'oxalate de calcium et où x peut être de préférence égal à 5, 15 ou 30.

### Exemple 4 : la paralysie familiale hyperkaliémique

C'est une maladie dite génétique qui consiste en accès de paralysies provoquées par un excès intracellulaire et aussi extracellulaire de potassium. Son traitement, dans l'état de l'art antérieur consiste en l'administration de diurétiques inhibiteurs de l'anhydrase carbonique. Les généticiens localisent l'anomalie génétique sur le chromosome 17. L'invention se donne pour objet de traiter cette maladie par le composé RxCH dans lequel R est de préférence le potassium ou un sel de potassium par exemple : le phosphate dipotassique (ou Kalium phosphoricum, produit connu et actif en homéopathie) et où x peut être de préférence égal à 15 à 30.

### Exemple 5 : L'hémochromatose

L'hémochromatose est une maladie dite génétique due à l'accumulation intra-cellulaire de fer. Son traitement dans l'état de l'art antérieur, consiste en des saignées et en l'administration d'un chélateur du fer le Desféral®. La présente invention a pour objet de traiter l'hémochromatose par le composé RxCH, dans lequel R est de préférence le fer, et dans lequel x est de préférence égal à 15 ou 30.

### Exemple 6 : La maladie de Wilson

La maladie de Wilson est une maladie due à l'accumulation intracellulaire de cuivre. Son traitement dans l'état de l'art antérieur consiste en l'administration d'un chélateur du cuivre : la Pénicillamine. La présente invention a pour objet de traiter la maladie de Wilson par le composé RxCH dans lequel R peut être de préférence le cuivre et où x peut être de préférence égal à 5, 15, ou 30.

### Exemple 7 : La maladie d'Alzheimer

C'est une maladie dans laquelle on a constaté un excès d'aluminium. L'invention concerne l'utilisation du composé RxCH pour traiter la maladie d'Alzheimer. R peut être de préférence l'aluminium, ou 1 autre sel ou dérivé de l'aluminium et x peut être de préférence égal à 5, 15, ou 30.

### B) Les maladies par carence intracellulaire :

### Exemple 8 : La tétanie

Il s'agit d'une maladie consistant en spasmes musculaires provoqués par une carence intracellulaire en calcium ou en magnésium. Lorsqu'il s'agit d'une carence intracellulaire en magnésium, cette maladie est dite génétique et son traitement dans l'état de l'art antérieur consiste en l'administration de magnesium. L'Invention dans ce cas concerne l'utilisation du composé RxCH dans lequel R est le phosphate dimagnesien (Magnesia phosphorica) et où x est de préférence égal à 4CH, 6DH, 5CH ou 7CH pour traiter la forme de tétanie dite génétique.
Dans les autres cas de tétanie R peut être le phosphate tricalcique : Calcarea phosphorica de préférence, ou d'autres sels ou corps chimiques.

### Exemple 9 : le rachitisme vitamino-résistant

Il s'agit d'une forme particulière de rachitisme chez l'enfant. La présente invention consiste à traiter cette affection par le composé RxCH où R peut être de préférence :
- le calcium ou des sels minéraux dérivés du calcium
- le calcaire d'huître ou Calcarea carbonica
- le phosphate tricalcique.

### Exemple 10 : La paralysie familiale hypokaliémique

C'est une maladie dite génétique qui consiste comme l'exemple 4 en accès de paralysies, mais qui sont ici provoquées par une carence intracellulaire et aussi extracellulaire en potassium et que l'invention a pour objet de traiter par le composé RxCH où R peut être de préférence à titre indicatif le potassium ou 1 sel de potassium par exemple le phosphate dipotassique ou Kalium phosphoricum produit actif bien connu en homéopathie et où x peut être de préférence égal à 5.

### Exemple 11 : anémie par carence en fer intra-cellulaire.

Il s'agit d'une affection peu connue caractérisée en ce que les globules rouges sont petits et l'hématocrite basse, et qui se manifeste par des symptômes d'anémie. L'invention consiste à traiter cette forme d'anémie par le composé RxCH où R est des préférence le fer et ou x est de préférence égal à 5.

### C) Le groupe des maladies qui sont reliées que très secondairement aux anomalies des systèmes transporteurs qui les ont engendrées :

### Exemple 12 : la polyarthrite rhumatoide

Les études cliniques pratiquées par l'inventeur ont montré que cette maladie auto-immune d'origine antérieurement indéterminée était due à une anomalie des systèmes transporteurs péricellulaires vis-à-vis du sulfure noir d'antimoine avec carence intra-cellulaire en sufure noir d'antimoine. L'invention a pour objet le traitement de cette affection par le composé RxCH dans lequel R est de préférence le sulfure noir d'antimoine et où x est de préférence égal à 4, 5 ou 7.

L'invention concerne également le traitement d'autres affections auto-immunes ne s'accompagnant pas de polyarthrite par le même composé RxCH où R est de préférence le sulfure noir d'antimoine. L'inventeur a observé un cas d'auto-immunisation anti-plaquettaire entrant dans ce cadre.

### Exemple 13 : Le lupus érythémateux disséminé

L'invention concerne le traitement de cette affection auto-immune par le composé RxCH dans lequel R est de préférence l'or et où x est de préférence égal à 4, 5 ou 7.

### Exemple 14 : La sclérose latérale amyotrophique

Les études cliniques pratiquées par l'inventeur ont montré que cette maladie incurable pouvait être une maladie auto-immune par anomalie des systèmes transporteurs péricellulaires vis-à-vis du phosphore. Aussi l'invention permet l'obtention d'un médicament destiné au traitement de cette maladie par le composé RxCH où R peut être de préférence le phosphore, ou un sel dérivé du phosphore et où x peut être de préférence égal à 5, 15 ou 30. La sclérose en plaque est une maladie très voisine, bénéficiant du même type de traitement par le même composé RxCH où R peut être de préférence le phosphore ou un sel dérivé du phosphore.

### Exemple 15 : L'hyperthyroïdie

Les études cliniques pratiquées par l'inventeur ont montré que cette maladie est due à un dérèglement des systèmes transporteurs péricellulaires vis-à-vis du NaCl, avec carence intracellulaire en NaCl. On remarquera que la mucoviscidose qui comporte un excès intracellulaire en NaCl constitue une entité tout-à-fait différente. L'Invention permet l'obtention d'un médicament destiné au traitement de l'hyperthyroïdie par le composé RxCH dans lequel R est de préférence le NaCl dans lequel x est de préférence égal à 6DH, 4CH, 5CH ou 7CH.

### D) Le groupe des maladies diverses :

### Exemple 17 : maladies diverses

On citera enfin comme autres exemples de maladies métaboliques à titre non limitatif les affections suivantes. La maladie de Refsum ou R peut être l'acide Phytanique, la maladie de Charcot Marie Tooth et de Déjerine Sottas, la chorée de Huntington où R peut être le zinc, la maladie de Thevenard, la maladie de Friedrich, l'hérédo ataxie cérébelleuse de Pierre Marie, la paralysie familiale de Strumpell Lorrain, la dystasie aréflexie de Roussy Lévis, les dyslipidoses, les Retards mentaux idiopathiques de l'enfant, l'autisme.
**Sont rapportés ci-après quelques observations cliniques de cas de patients traités par des composés RxCH constitutifs d'un médicament dont l'invention permet l'obtention.**

### CAS N° 1

Malade âgée de 72 ans, de sexe féminin, présentant un teint très mat et un léger tremblement des deux membres supérieurs. Un examen demandé à titre systématique pour un épisode d'asthénie montre un fer sérique anormalement élevé à 173 µg/L soit 31 µmol le 11 octobre, sans que l'on puisse parler d'hémochromatose. On prescrit Ferrum metallicum 15CH à raison d'une dose chaque dimanche à partir du 17 octobre et pendant une durée d'un mois.
- Le 16 novembre c'est-à-dire en fin de traitement un nouveau dosage de fer sérique est effectué. Il est descendu à 103,41 µg soit 18,50 µmol. La patiente n'a souffert d'aucune hémorragie et la baisse très significative du fer sérique de
   . 31 µmol à 18,5 µmol.
   . 173 µg/L à 103 µg/L
   est due à l'administration de Ferrum metallicum qui a provoqué l'élimination du fer hors de l'organisme.
   L'état général est meilleur. Le tremblement est moins prononcé.
- Une nouveau dosage de fer sérique est demandé 2 mois 1/2 plus tard, fin janvier c'est-à-dire 2 mois 1/2 après l'arrêt du traitement de un mois par Ferrum metallicum 15CH. Le fer sérique est remonté sans toutefois avoir atteint son taux de départ. Il est à :
   . 25 µmol/L soit 139,75 µg/L
   ce qui prouve que la malade est porteuse d'une anomalie métabolique qui lui donne un fer spontanément élevé ou qu'elle absorbe du fer d'une façon anormale, ce qui n'est pas le cas : son alimentation excluant cette possibilité. Le 31 janvier on represcrit Ferrum metallicum 15CH à raison d'une dose chaque dimanche pendant un mois sans refaire de dosage en fin de traitement. On note pendant cet hiver une amélioration de l'état général et une absence de l'épisode de bronchite qui survenait tous les hivers d'une façon habituelle et qui durait environ un mois. Au mois de juillet suivant, l'on refait un dosage de fer sérique qui est à
   . 26,80 µmol/L soit 149,81 µg/L
   soit stationnaire.
   Ce cas illustre les théories et expériences sur les cobayes, à savoir qu'il y a élimination hors de l'organisme du produit dont on a administré la dilution homéopathique. Cette élimination concerne un appel d'ions hors des cellules, le stockage du fer étant intracellulaire. De plus la malade citée ici qui a une tendance spontanée à avoir un fer sérique élevé pourrait bien être porteuse d'une forme hétérozygote, mineure et très atténuée d'hémochromatose, et ce cas concernerait aussi les théories d'eveloppées dans le cadre de l'invention sur cette affection.

### CAS N° 2

Malade de sexe féminin âgée de 44 ans, présentant une hyperthyroïdie due à une hyperplasie thyroïdienne plurinodulaire et une rhinite allergique matinale. Sur la cartographie, il y avait une hypertrophie du lobe droit avec un nodule froid. Les examens biologiques effectués à l'issue de la première consultation montraient une hyperthyroïdie avec
- le 19 février: TSH 0,02
T4L 35
Les taux normaux de TSH sont de 0,2 à 4 et de T4l 10 à 25. La TSH est anormalement basse et la T4 libre anormalement élevée. La malade reçoit un traitement d'antithyroïdien de synthèse, seul avec du Basdène® à compter du 22 février aux doses croissantes de 1cp le 1er jour, 2cp le 2ème jour, puis 3cp par jour.
- Le 3 mars: TSH : 0,02
T4l à 36,8

Donc quelques jours après le début du traitement de Basdène® l'hyperthyroïdie est restée identique et même s'est légèrement aggravée. Le 7 mars la malade est revue en consultation et à la place du Basdène®, on prescrit du Néomercazole® aux doses de 3cp 3 fois par jour
- le 17 mars: TSH : 0,02
T4l : 33,8

Le taux d'Hormone thyroïdienne après 10 jours de Néomercazole® est toujours aussi élevé, sans aucune amélioration.

Le 20 mars la malade est revue à nouveau et on rajoute au traitement de néomercazole, pour la rhinite allergique :
Natrum muriaticum 6DH 3 cp/1 pendant 7 jours puis
Natrum muriaticum 4CH 3 granules/1 pendant 15 jours.
   - Le 13 avril: T4l : 25,1

Le taux d'hormone thyroïdienne pour la première fois est redevenu normal et l'abondance de la rhinite allergique a diminué des 3/4.

Pour savoir si c'est le Natrum muriaticum ou le Néomercazole® qui a normalisé les fonctions thyroïdiennes, on arrête brutalement le Néomercazole®, ce qui doit avoir pour effet si c'est le Néomercazole® qui a fait baisser la T4l, de la faire remonter immédiatement à 30 et l'on poursuit la prise régulière de Natrum muriaticum 4CH ceci le 13 avril.

Le 27 avril : T4l : 26,9 donc toujours normal, ce qui est la preuve que c'est bien le Natrum muriaticum et non pas le Néomercazole® qui a guérit l'hyperthyroïdie et normalisé le taux de T4l. Les doses précises de Natrum muriaticum qui ont été effectivement prises sont de
- Natrum muriaticum 6DH : 3 cp/jour du 20 mars au 11 avril puis Natrum muriaticum 4CH : 3 granules/jour du 11 au 28 avril. Le 28 avril la malade est revue en consultation et on modifie encore la dose prescrite de Natrum muriaticum que l'on augmente à raison de Natrum muriaticum 5CH : 3 granules par jour.
Le Néomercazole® n'a bien entendu jamais été repris. Ce changement de doses de Natrum muriaticum a pour effet de faire baisser encore l'hormone thyroïdienne :
- le 27 avril: T4l : 26,9
- le 10 juin: T4l : 18,6
On poursuit toujours à partir de ce moment là la dose suivante de Natrum muriaticum 5CH : 3 granules par jour.
- Le 1^{er} août on constate une disparition de la rhinite allergique
- Entre temps le 10 juillet, la malade a été opérée de son goitre hyperplasique plurinodulaire comportant des nodules froids que l'on suspectait de néoplasie malgré les nombreuses ponctions pratiquées qui n'avaient jamais rien montré d'alarmant. Sur la pièce opératoire il n'y avait aucun cancer. Cette opération se solde par les 1^{er} août une T4l à 13,8 vers la limite inférieure de la normale cette fois-ci (la normale étant de 10 à 25). On pourrait craindre une évolution vers l'hypothyroïdie. Il n'est est rien :
   - -le 9 octobre: TSH : 2,14
   T4l : 16,7

Les fonctions thyroïdiennes sont donc normales. La rhinite allergique a disparu, le Natrum muriaticum 5CH à raison de 3 granules par jour, étant toujours poursuivi. En conclusion, cet exemple illustre le fait que l'hyperthyroïdie est bien une maladie par carence intracellulaire en NaCl, et que la prescription de NaCl 5CH en agissant sur le rétablissement d'un fonctionnement normal des systèmes transporteurs, normalise d'une façon quasi immédiate les métabolismes hormonaux thyroïdiens qui étaient perturbés.
Un autre métabolisme perturbé redevient normal : c'est la production d'anticorps anormaux de type IgE responsables de la rhinite allergique qui cesse complètement au bout de quelques mois de traitement, et qui avait commencé à diminuer et à s'améliorer dès les premières prises de remède homéopathique.

### CAS N°3

Enfant âgé de 7 ans, de sexe féminin, atteinte d'une polyarthrite chronique juvénile, maladie auto-immune qualifiée de génétique.
Après examen du cas clinique il apparaît que les symptômes homéopathiques correspondent à une carence intracellulaire en sulfure noir d'antimoine. Lors de la première consultation la malade présente une atteinte polyarticulaire surtout localisée aux coudes et aux genoux nécessitant une brise quotidienne de Naprosyne® et des séances de kinésithérapie. Le Naprosyne® est pris à la dose de 1cp à 250mg par jour et ne suffit plus à juguler les symptômes car la maladie s'aggrave. C'est ce qui motive la consultation. On rajoute au Naprosyne® : Antimonium crudum 5CH 3 à 5 granules 1 fois par jour, ceci dès le 27 décembre. L'état articulaire après cette date, s'améliore si nettement que les doses de Naprosyne® qu'il aurait fallu agmenter sont diminuées ; au début du mois d'avril suivant, le Naprosyne® est complètement arrêté et il est pris de temps en temps un peu de Voltarène®. Au mois de juillet suivant, le bras droit qui était déformé s'est complètement redressé. L'articulation du coude est redevenue normale. Les symptômes de polyarthrite sont passés. Bien entendu, pendant tout ce temps l'enfant a pris chaque jour 3 à 5 granules d'Antimonium crudum 5CH. A partir du mois de juillet, les anti-inflammatoires sont arrêtés et le traitement d'Antimonium crudum 5CH est toujours poursuivi. Il le sera plusieurs années.

### En conclusion

Ce cas illustre le fait qu'une maladie auto-immune, dite génétique a pu être guérie par le rétablissement du fonctionnement normal des systèmes transporteurs péricellulaires.
Ici la polyarthrite chronique rhumatoïde se révèle être une maladie par carence intracellulaire en sulfure noir d'antimoine.
En conclusion de ce cas et du cas précédent, l'on vérifie la théorie développée dans le cadre de l'invention qui affirme que la correction des anomalies de fonctionnement des systèmes transporteurs péricellulaires normalise secondairement les métabolismes perturbés.

### CAS N°4

Chien de type berger allemand de 3 ans 1/2 atteint d'une polyarthrite auto-immune du chien, maladie qui correspondrait en pathologie humaine au lupus érythémateux disséminé. Les symptômes de cette affection sont un blocage complet de l'arrière train et une fièvre à 40°, et même 41°. Le traitement de médecine classique débute le 8 juillet 1986 et consiste en injection de corticoïdes, à raison de
Solumédrol® 20 mg matin et soir en IM sous couverture antibiotique maintenue une dizaine de jours. Puis les corticoïdes sont diminués progressivement. Le Solumédrol® est remplacé par du Cortancyl® 5mg : 2cp par jour.
Les symptômes homéopathiques correspondaient à une carence intracellulaire en or et à la prescription d'Aurum métallicum. En août de la même année, on rajoute au Cortancyl® un remède homéopathique. Mais l'on se trompe de remède homéopathique, au lieu d'Aurum métallicum, on fait prendre au chien Mercurius solubilis : 3 à 5 granules par jour. A partir du début septembre croyant à une guérison, on diminue progressivement le Cortancyl®. La maladie rechute immédiatement et l'on doit reprendre le traitement de Solumédrol® 20mg matin et soir en IM pendant une dizaine de jours suivi à nouveau du Cortancyl® 5mg : 2cp/jour en supprimant bien entendu le Mercurius solubilis.
Le 18 Mars de l'année suivante, alors que l'on a pu réduire les doses de Cortancyl® 5mg à 1cp/jour on rajoute cette fois-ci Aurum métallicum 5CH 3 à 5 granules par jour, prescription enfin exacte. Deux mois plus tard le Cortancyl® a pu être diminué avec succès et il n'est plus pris qu'à la dose de 1cp à 5mg une fois par semaine chaque lundi. Pendant quelques jours, au mois d'avril, on oublie de donner Aurum métallicum 5CH, ce qui provoque un matin de l'aphonie (perte complète de la voix et de l'aboiement), et par un petit début de raideur de l'arrière-train. On reprend immédiatement le traitement d'Aurum métallicum 5CH à raison de 3 granules plusieurs fois par jour (3 fois) accompagné de Ledum palustre 7CH : 3 granules 2 fois par jour, ceci pendant 3 jours. Par la suite l'on poursuit le traitement aux doses suivantes : Aurum métallicum 5CH : 5 granules par jour
Aurum métallicum 7CH : 3 granules par jour
Le dernier 1/2 comprimé de Cortancyl® 5mg a été donné le 28 septembre 1987 et depuis les corticoïdes ont été arrêtés définitivement. Le traitement homéopathique a été poursuivi aux doses indiquées ci-dessus jusqu'en août 1989 où il sera arrêté définitivement. Il n'y aura jamais de rechute.

### En conclusion

Ce cas illustre et confirme les théories de l'invention, et montre que cette forme de polyarthrite auto-immune du chien est due à une carence intracellulaire en or, et qu'il est probable que la maladie humaine correspondante le lupus erythémateux disséminé soit due aussi à une carence intracellulaire en or.
Il montre aussi qu'un traitement à l'aide d'un médicament dont l'invention permet l'obtention rétablit à long terme le fonctionnement normal des systèmes transporteurs péricellulaires sans possibilité de rechute et que le rétablissement de ce fonctionnement normal des systèmes transporteurs supprime les anomalies métaboliques des cellules à savoir ici la production d'anticorps anormaux.

## Revendications

1. Utilisation d'un produit homéopathique de formule générale RxCH, où R désigne un principe actif et où xCH représente sa dilution homéopathique, notamment Hahnemanienne ou Korsakowienne, dans ledit produit pour l'obtention d'un médicament destiné à provoquer l' élimination hors des cellules qui le contiennent d'un composé de nature identique à celle dudit principe actif, lorsque cette élimination a pour effet de rétablir le fonctionnement normal des systèmes transporteurs péricellulaires peturbés.

2. Utilisation selon la revendication 1, caractérisée en ce que le composé à éliminer est un composé minéral et que son élimination a pour effet le rétablissement du fonctionnement normal d'un canal ionique.

3. Utilisation selon la revendication 1 caractérisée en ce que le composé à éliminer est un toxique ou une partie d'un toxique.

4. Utilisation selon la revendication 1 pour l'obtention d'un médicament destiné à traiter les maladies trouvant leur origine dans une erreur métabolique consistant soit en une accumulation intracellulaire d'un composé de formule simple ou complexe, soit en une carence intracellulaire d'un tel composé.

5. Utilisation selon la revendication 4 caractérisée en ce que l'erreur métabolique a pour conséquence la production de métabolites anormaux, notamment d'anticorps anormaux tels que des auto-anticorps.

6. Utilisation selon l'une des revendications 4 et 5, caractérisée en ce que l'erreur métabolique est déterminée génétiquement.

## Claims

1. Use of a homeopathic product of the general formula RxCH, in which R is an active principle and in which xCH is its homeopathic dilution, especially hahnemannian or korsakowian homeopathic dilution, in said product, for the preparation of a drug intended for causing the elimination, from the cells which contain it, of a compound identical in nature to said active principle when the effect of this elimination is to restore normal function to the perturbed pericellular transport systems.

2. Use according to claim 1, characterized in that the compound to be eliminated is a mineral compound and that the effect of its elimination is to restore normal function to an ion channel.

3. Use according to claim 1, characterized in that the compound to be eliminated is a poison or part of a poison.

4. Use according to claim 1 for the preparation of a drug intended for treating diseases caused by a metabolic error consisting either of an intracellular accumulation of a compound of simple or complex formula, or of an intracellular deficit of such a compound.

5. Use according to claim 4, characterized in that the consequence of the metabolic error is the production of abnormal metabolites, especially abnormal antibodies such as autoantibodies.

6. Use according to one of claims 4 and 5, characterized in that the metabolic error is genetically determined.

## Patentansprüche

1. Verwendung eines homöopathischen Präparats mit der allgemeinen Formel RxCH, worin R einen Wirkstoff bezeichnet und xCH dessen homöopathische, namentlich Hahnemannsche oder Korsakowsche, Verdünnung im besagten Präparat, zum Zwecke des Erhalts einer Arznei, die dazu bestimmt ist, die Elimination einer Verbindung, die von gleicher Art ist wie der besagte Wirkstoff, aus den die Verbindung enthaltenden Zellen herbeizuführen, wenn die Elimination der Verbindung die Wiederherstellung der normalen Funktion der gestörten perizellulären Transportsysteme zur Folge hat.

2. Verwendung nach Anspruch 1, gekennzeichnet dadurch, daß die zu eliminierende Verbindung eine anorganische Verbindung ist und deren Elimination die Wiederherstellung der normalen Funktion eines Ionenkanals zur Folge hat.

3. Verwendung nach Anspruch 1, gekennzeichnet dadurch, daß die zu eliminierende Verbindung ein Giftstoff oder der Teil eines Giftstoffs ist.

4. Verwendung nach Anspruch 1 zwecks Erhalts einer Arznei zur Behandlung von Krankheiten, deren Ursache ein Stoffwechselirrtum ist, der aus der intrazellulären Ansammlung einer Verbindung mit einfacher oder komplexer Zusammensetzung oder dem intrazellulären Mangel einer solchen Verbindung besteht.

5. Verwendung nach Anspruch 4, gekennzeichnet dadurch, daß der Stoffwechselirrtum die Bildung anormaler Metaboliten, namentlich anormaler Antikörper wie Autoantikörper, zur Folge hat.

6. Verwendung nach einem der Ansprüche 4 und 5, gekennzeichnet dadurch, daß der Stoffwechselirrtum genetisch bedingt ist.
